# FASCICULE DE BREVET EUROPEEN

(11) **EP 2 335 567 B1**
(45) Date de publication et mention de la délivrance du brevet: **25.04.2018**
(21) Numéro de dépôt: 10354091.0
(22) Date de dépôt: 16.12.2010
(51) Int. Cl.: A61B 5/00

(54) **Dispositif et procédé d'évaluation de la perte hydrique d'un individu ou d'un animal par sudation**
Vorrichtung und Verfahren zur Abschätzung des Wasserverlusts einer Person oder eines Tieres durch Schwitzen
Device and method for assessing the water lost by a person or an animal through sweating

(30) Priorité: 21.12.2009 FR 0906204
(43) Date de publication de la demande: 22.06.2011
(73) Titulaire: Commissariat à l'Énergie Atomique et aux Énergies Alternatives, 75015 Paris (FR)
(72) Inventeur: Revol-Cavalier, Frédéric, 38180 Seyssins (FR)
(74) Mandataire: Dubreu, Sandrine

(56) Documents cités:
- EP-A1- 0 421 625
- WO-A1-2005/120333
- JP-A- 2002 263 072
- US-A- 3 350 941
- US-A- 4 655 076
- US-A- 4 965 698

## Description

### Domaine technique de l'invention

L'invention concerne un dispositif et un procédé d'évaluation de la perte hydrique d'un individu ou d'un animal par sudation.

### État de la technique

De nombreux travaux ont montré l'intérêt d'une bonne hydratation chez les individus notamment lors d'activités physiques sportives ou chez les personnes fragiles comme les nourrissons ou les personnes âgées. La perte hydrique due à la transpiration ou le manque d'hydratation peut entraîner l'apparition de troubles physiologiques, par exemple, la perte de poids, des troubles de la concentration, une fatigue importante ou des vertiges. Pour les cas de déshydratation les plus sévères, des pertes de facultés intellectuelles ou de troubles physiologiques pouvant entraîner la mort de l'individu ou de l'animal peuvent également survenir.

La sudation est le principal moteur de la régulation thermique du corps humain et de certains animaux supérieurs. Lorsque la sudation devient insuffisante pour refroidir le corps, la température corporelle interne augmente. Pour éviter la déshydratation ou la surhydratation, une approche consiste à suivre la température interne du corps selon des techniques classiques de mesure de température du corps. Les mesures externes de la température corporelle, par exemple axillaires, ne sont pas suffisamment précises et rapides pour un tel suivi. D'autres part, les mesures internes de la température corporelle, par exemple buccales, rectales ou auriculaires, ont un usage restreint, limité au laboratoire et au milieu médical, et sont peu adaptées à un usage en continu et en temps réel.

Une autre approche consiste à évaluer la perte hydrique par sudation c'est-à-dire la quantité de sueur transpirée sur un temps donné. Cette évaluation est, classiquement, pratiquée par pesée sur une balance différentielle. L'individu est pesé à plusieurs reprises, tout au long d'un exercice physique ou lors du contrôle pour permettre de calculer le rapport entre la perte hydrique et le poids de l'individu. Ce rapport est caractéristique du taux de déshydratation de l'individu et, par conséquent, de son état d'hydratation. Néanmoins, cette solution est difficile à mettre en oeuvre hors laboratoire ou hors milieu médical. Cette solution est précise et sensible, mais ne demeure applicable qu'en laboratoire et ne peut convenir à des applications nomades.

Des travaux récents ont proposé des dispositifs permettant d'évaluer la perte hydrique d'un individu à partir de la mesure du taux d'humidité au voisinage de la peau de l'individu. À titre d'exemple, les documents GBA-2452258, WO2005120333, US-A-4965698, JP-A-2002263072 et US-A-3350941 décrivent des dispositifs appliqués à la surface de la peau d'un individu, aménageant un espace de mesure au-dessus de la peau contenant un capteur d'humidité, pour mesurer l'humidité contenue dans l'atmosphère et représentative de la sueur sécrétée.

### Objet de l'invention

L'invention a pour but un dispositif embarqué sur un individu ou un animal, précis et fiable, permettant d'évaluer, en temps réel et en continu, la perte hydrique par sudation de l'individu ou l'animal.

Selon l'invention, ce but est atteint par un dispositif d'évaluation de la perte hydrique d'un individu ou d'un animal par sudation et un procédé utilisant un tel dispositif selon les revendications annexées.

En particulier, ce but est atteint par un dispositif muni de :
- un support souple, apte à pouvoir s'adapter à la forme du corps sur lequel il est appliqué, comportant une face de mesure et une face de contact destinée à être appliquée sur la peau de l'individu ou de l'animal,
- un élément gonflant par absorption de sueur et solidaire du support, ledit élément gonflant étant agencé pour être en contact direct ou en communication fluidique avec la peau et,
- des moyens de mesure de l'augmentation d'au moins une dimension de l'élément gonflant, ladite augmentation étant provoquée par absorption de ladite sueur.

### Description sommaire des dessins

D'autres avantages et caractéristiques ressortiront plus clairement de la description qui va suivre de modes particuliers de réalisation de l'invention donnés à titre d'exemples non-limitatifs et représentés aux dessins annexés, dans lesquels :
- La figure 1 représente, schématiquement et en coupe, un mode de réalisation particulier d'un dispositif d'évaluation de la perte hydrique d'un individu ou d'un animal par sudation selon l'invention.
- La figure 2 représente, schématiquement et en coupe, une étape d'un mode de réalisation d'un procédé utilisant le dispositif selon la figure 1.
- Les figures 3 à 5 représentent, schématiquement et en vue de dessus, des modes particuliers de réalisation d'un dispositif selon les figures 1 et 2.
- La figure 6 représente, schématiquement et en coupe, un second mode particulier de réalisation d'un dispositif selon l'invention.
- La figure 7 représente, schématiquement et en coupe, une étape d'un mode de réalisation d'un procédé utilisant un dispositif selon la figure 6.
- La figure 8 représente, schématiquement et en coupe, une variante du dispositif selon la figure 7.
- La figure 9 représente, schématiquement et en coupe, un troisième mode particulier de réalisation d'un dispositif selon l'invention.
- La figure 10 représente, schématiquement et en coupe, une étape d'un mode de réalisation d'un procédé utilisant un dispositif selon la figure 9.
- La figure 11 représente, schématiquement et en coupe, une variante du dispositif selon la figure 10.
- La figure 12 représente, schématiquement et en coupe, un montage opératoire pour l'étalonnage d'un dispositif selon l'invention.
- La figure 13 représente le schéma électronique du montage selon la figure 12.
- La figure 14 est un graphique représentant la tension mesurée à partir du montage de la figure 12 et selon le schéma électronique de la figure 13 en fonction du volume d'eau absorbée.

### Description de modes particuliers de réalisation

L'invention a pour objet un dispositif destiné à une utilisation embarquée sur tout individu et animal possédant des glandes sudoripares et apte à transpirer. Le dispositif permet d'évaluer la perte hydrique de l'individu ou de l'animal par sudation, à partir d'une estimation de la quantité de sueur transpirée par l'individu ou l'animal. L'utilisation du dispositif est réalisée, de préférence, en continu et en temps réel, pour une lecture directe de l'évaluation de la perte hydrique.

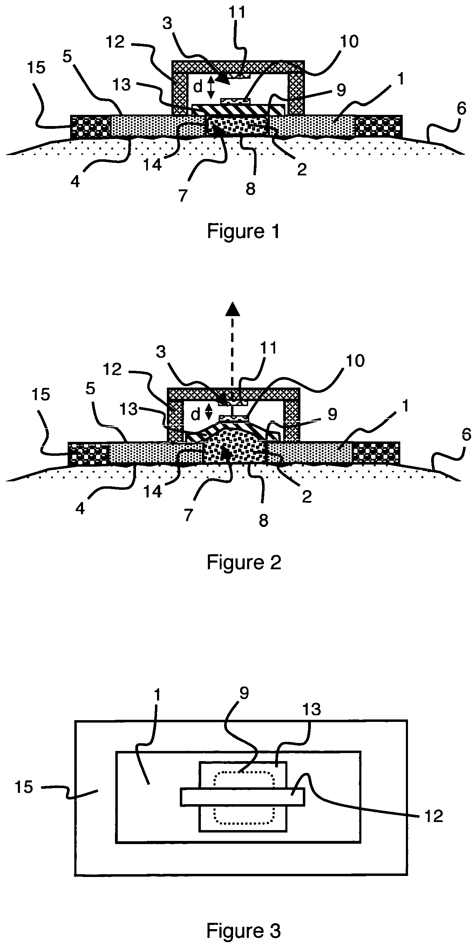

Selon un premier mode de réalisation particulier représenté aux figures 1 à 5, un dispositif est muni d'un support 1, d'un élément gonflant 2 et de moyens de mesure 3 de l'augmentation d'au moins une dimension de l'élément gonflant 2.

Le support 1 comporte une face de contact 4 et une face de mesure 5. La face de contact 4 est destinée à être appliquée sur la peau 6 de l'individu ou de l'animal. La mesure de l'augmentation d'au moins une dimension de l'élément gonflant 2 est réalisé du côté de la face de mesure 5.

Le support 1 est constitué, par tout matériau connu suffisamment souple pour pouvoir s'adapter à la forme du corps sur lequel il est appliqué et ayant une tenue mécanique suffisante pour supporter les éléments constitutifs du dispositif. En outre, le support 1 est inerte vis-à-vis de la sueur et, de préférence, dermatologiquement acceptable.

L'élément gonflant 2 est solidaire du support 1 et apte à gonfler par absorption de sueur. L'élément gonflant 2 est agencé pour être en contact direct ou en communication fluidique avec une zone localisée de la peau 6, recouverte par le dispositif, de sorte que la sueur émise par sudation au niveau de cette zone est absorbée par l'élément gonflant 2. On entend par communication fluidique le fait que la sueur transpirée par la peau 6 est véhiculée par tout procédé connu de la peau 6 vers l'élément gonflant 2. L'élément gonflant 2 absorbe la sueur émise sous forme liquide. De la sueur sous forme vapeur, à proximité de la surface de la zone localisée à absorber, peut également être absorbée par l'élément gonflant 2.

L'élément gonflant 2 comporte, avantageusement, un polymère hydrophile. Le polymère hydrophile est, de préférence, choisi parmi les polymères hydrophiles synthétiques, les polysaccharides naturels, les polysaccharides hémi-synthétiques et les dérivés de la cellulose et leurs sels.

À titre d'exemple, les polysaccharides naturels peuvent être des alginates, de la gomme de xanthane, de guar, arabique ou de caroube.

Les polysaccharides hémi-synthétiques sont, de préférence, choisis parmi les dérivés de la cellulose, par exemple, parmi la méthylhydroxyéthylcellulose, la carboxyméthylcellulose et ses sels tels que la carboxyméthylcellulose sodique ou la carboxyméthylcellulose calcique, l'hydroxypropylcellulose, l'hydroxypropylméthylcellulose et les mélanges d'hydroxypropylcellulose et d'hydroxypropylméthylcellulose.

Le polymère hydrophile peut également être choisi parmi les polyvinylpyrrolidones, les polymères d'acides aminés comme les polylysines et, avantageusement, parmi les polymères dérivés des acides acrylique et méthylacrylique et leurs sels. Le polymère hydrophile est, de préférence, un polyacrylate ou un sel de polyacrylate.

L'élément gonflant 2 peut être constitué par un seul polymère hydrophile cité ci-dessus ou par un mélange de plusieurs d'entre eux.

Afin de favoriser une augmentation rapide du volume de l'élément gonflant 2, avec les polymères hydrophiles précédemment cités, l'élément gonflant 2 peut comporter des agents de stimulation du gonflement. Les agents de stimulation du gonflement favorisent l'hydratation des structures polymères de l'élément gonflant 2 et/ou diminuent les interactions entre les structures polymères de l'élément gonflant 2 et les constituants de la sueur. On peut aussi utiliser à cet effet des diluants hydrophiles tels que le lactose, le mannitol, le sorbitol et la cellulose microcristalline. On peut également utiliser des substances qui favorisent la mouillabilité de la structure polymère de l'élément gonflant 2 comme le laurylsulphate de sodium, le rinoléate de sodium, le téradécylsulphate de sodium, le dioctylsulphosulphonate de sodium, le cétomagrocol, le poloxamère, les polysorbates ou tout autre tensioactif dermatologiquement acceptable.

L'élément gonflant 2 peut également comporter, avantageusement, des fibres textiles comme du coton, pour répartir uniformément la sueur au sein de l'élément glonflant 2 et assurer un gonflement uniforme du polymère hydrophile.

Les moyens de mesure 3 sont, de préférence, des capteurs ou des détecteurs disposés au niveau de la face de mesure 5 afin de déterminer l'augmentation d'au moins une dimension de l'élément gonflant 2 provoquée par absorption de sueur. L'élément gonflant 2 et les moyens de mesure 3 sont deux éléments distincts dans le dispositif, avec des fonctions différentes. L'élément gonflant 2 absorbe la sueur transpirée par la peau 6, majoritairement sous forme liquide, et les moyens de mesure 3 déterminent l'augmentation de volume de l'élément gonflant 2 provoquée par l'absorption la sueur transpirée.

Les moyens de mesure 3 sont, avantageusement, choisis parmi les photodétecteurs, les capteurs capacitifs, les capteurs inductifs, les capteurs mécaniques de position et les capteurs de type jauge de contrainte.

Les moyens de mesure 3 sont reliés, classiquement, à un système de traitement et d'affichage de données (non représenté). Les données sont, par exemple, stockées et/ou transmises par voie filaire ou non filaire à un circuit électronique, puis traitées et affichées par tout procédé connu.

Comme représenté aux figures 1 et 2, le support 1 comporte un trou traversant 7 débouchant sur la face de contact 4 et sur la face de mesure 5, respectivement, par une première ouverture 8 et une seconde ouverture 9. L'élément gonflant 2 est logé et contraint dans le trou traversant 7 de sorte que l'augmentation d'une dimension de l'élément gonflant 2 provoque la saillie de l'élément 2 hors du trou traversant 7. L'élément gonflant 2 fait saillie hors du trou traversant 7, principalement, du côté de la face de mesure 5 car le dépassement de l'élément gonflant 2 du côté de la face de contact 4 est entravé par la présence de la peau 6, la face de contact 4 étant appliquée sur la zone localisée de la peau 6 (figure 2).

Les moyens de mesure 3 sont, avantageusement, disposés au niveau de la face de mesure 5 pour mesurer l'augmentation de la dimension de l'élément gonflant 2 faisant saillie hors du trou traversant 7, du côté de la face de mesure 5. Ainsi, les moyens de mesure 3 permettent de mesurer la saillie de l'élément gonflant 2 hors du trou traversant 7 du côté de la face de mesure 5, cette saillie étant proportionnelle à la quantité de sueur absorbée.

Les moyens de mesure 3 sont, par exemple, constitués par un capteur capacitif. En particulier, les moyens de mesure 3 comportent un condensateur formé par une électrode mobile 10 et une électrode fixe 11 en regard de ladite électrode mobile 10. Les électrodes mobile et fixe, respectivement 10 et 11, sont de préférence métalliques. Un espace libre sépare l'électrode mobile 10 et l'électrode fixe 11 d'une distance d. L'électrode mobile 10 est disposée au-dessus de l'élément gonflant 2 et est solidaire dudit élément 2 de sorte que l'augmentation d'au moins une dimension de l'élément gonflant 2 provoque le déplacement de l'électrode mobile 10 vers l'électrode fixe 11.

Comme représenté à la figure 1, le dispositif peut comporter une armature 12 située sur la face de mesure 5. Les moyens de mesure 3 peuvent, en particulier, être disposés au-dessus de l'élément gonflant 2. Les moyens de mesure 3 peuvent être supportés par l'armature 12. En particulier, au moins une partie des moyens de mesure 3 est supportée par l'armature 12. Ainsi, l'électrode fixe 11 peut être supportée par l'armature 12 qui positionne l'électrode fixe 11 au-dessus de l'électrode mobile 10, elle-même située au-dessus de la seconde ouverture 9 (en haut à la figure 1).

La face de mesure 5 comporte des moyens de confinement 13 de l'élément gonflant 2 faisant saillie hors du trou traversant 7. Les moyens de confinement 13 peuvent être formés par une membrane déformable fixée à la face de mesure 5 et recouvrant au moins une partie de l'élément gonflant 2. Les moyens de confinement 13 sont, avantageusement, fixés au niveau d'au moins deux points de chevauchement des moyens de confinement 13 avec la face de mesure 5.

Comme représenté à la figure 2, lorsque le dispositif est appliqué sur une zone localisée de la peau 6, la première ouverture 8 est en regard de la peau 6 et obstruée par cette dernière. L'élément gonflant 2 est contenu par les parois latérales 14 du trou traversant 7. L'absorption de la sueur collectée au niveau de la zone de la peau 6 recouverte par l'élément gonflant 2 provoque l'expansion de l'élément gonflant 2 hors du trou traversant 7. L'élément gonflant 2 fait alors saillie à la surface de la face de mesure 5, par la seconde ouverture 9.

Comme représenté à la figure 2, l'électrode mobile 10 est solidaire de l'élément gonflant 2, pour que l'expansion de l'élément gonflant 2 par absorption de sueur induit le déplacement de l'électrode mobile 10 vers l'électrode fixe 11.

L'expansion de l'élément gonflant 2 provoque le rapprochement de l'électrode mobile 10 vers l'électrode fixe 11 et la diminution de la distance d entre les électrodes, respectivement 10 et 11. La variation de la distance d entraîne une variation de la capacité du condensateur formé par les deux électrodes fixe et mobile, respectivement 10 et 11. Ainsi, l'augmentation d'une dimension, par exemple la hauteur de l'élément gonflant 2 à la figure 2, est transcrite en une quantité physiquement mesurable : la capacité du condensateur. La capacité est alors proportionnelle au volume de sueur absorbé. Le suivi de la valeur de la capacité du condensateur permet de mesurer le volume de sueur absorbée en fonction du temps et, par conséquent, d'en déduire la perte hydrique de l'individu ou de l'animal.

L'électrode mobile 10 peut, avantageusement, être déposée sur la membrane constituant les moyens de confinement 13. L'élément de confinement 13 est disposé entre l'électrode mobile 10 et l'élément gonflant 2. L'électrode fixe 11 est située dans la trajectoire de l'expansion de l'élément gonflant 2 (flèche en pointillé à la figure 2).

Les moyens de confinement 13 accompagnent l'expansion de l'élément gonflant 2 hors du trou traversant 7, tout en le contenant pour circonscrire sa trajectoire et l'empêcher de se disperser. Les moyens de confinement 13 permettent ainsi de contenir l'élément gonflant 2 et de confiner la partie de l'élément gonflant 2 faisant saillie c'est-à-dire située hors du trou traversant 7.

Le support 1 comporte, avantageusement, des moyens de fixation 15 du dispositif sur la peau 6 ou sur le corps de l'individu ou de l'animal. Le support 1 peut, par exemple, comporter un ou plusieurs adhésifs.

Comme représenté à la figure 3, le dispositif peut être maintenu par un adhésif situé en périphérie du dispositif. L'adhésif maintient le dispositif sur la zone localisée de la peau 6 où la mesure doit être effectuée.

Selon une variante non représentée, les moyens de fixation 15 peuvent être constitués par des attaches destinées à ceinturer une partie du corps de l'individu ou de l'animal. Les attaches peuvent être tout organe de serrage mécanique connu apte à maintenir le dispositif autour d'une partie du corps de l'individu ou de l'animal. On peut citer, à titre d'exemple, les bandes de type Velcro®, les lanières, les bracelets ou les ceintures dorsales.

Selon une autre variante non représentée, une partie ou la totalité de la face de contact 4 peut être adhésive.

Comme représenté à la figure 3, les moyens de confinement 13 sont, de préférence, disposés au-dessus de l'élément gonflant 2, pour recouvrir la seconde ouverture 9 et une partie du support 1 en périphérie de la seconde ouverture 9. L'armature 12 peut avoir une forme d'arche prenant appui de part et d'autre de la seconde ouverture 9.

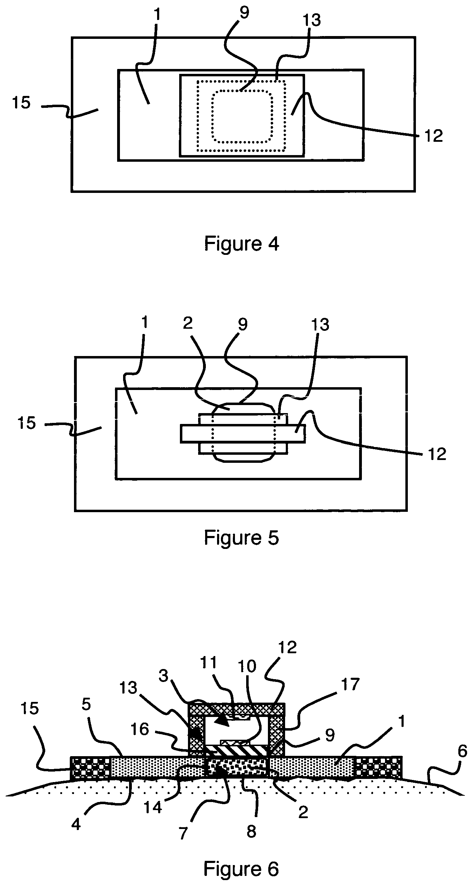

Selon une variante représentée à la figure 4, l'armature 12 peut être formée par un capot prenant appui sur toute la périphérie de la seconde ouverture 9 et crée alors un espace clos enfermant les moyens de mesure 3.
Selon une autre variante représentée à la figure 5, les moyens de confinement 13 ne couvrent qu'une partie de la seconde ouverture 9, laissant une partie de l'élément gonflant 2 non recouverte. La membrane formant les moyens de confinement 13 est fixée uniquement de part et d'autre de la seconde ouverture 9 (à droite et à gauche à la figure 5).

Selon un mode de réalisation particulier, un procédé d'évaluation de la perte hydrique d'un individu ou d'un animal par sudation comporte l'application du dispositif d'évaluation décrit ci-dessus sur une zone localisée de la peau 6 de l'individu ou de l'animal, la face de contact 4 étant positionnée en regard de la peau 6 et l'élément gonflant 2 étant en contact ou en communication fluidique avec la peau 6 (figure 1). Tout en maintenant le dispositif en place, on mesure ensuite l'augmentation d'au moins une dimension de l'élément gonflant 2 au cours du temps grâce aux moyens de mesure 3, par tout procédé connu (figure 2).

On mesure, avantageusement, l'augmentation d'une seule dimension de l'élément gonflant 2 au cours du temps. Cette dimension peut être la hauteur, la profondeur, la longueur ou la largeur.

Néanmoins, la mesure peut également être réalisée sur deux dimensions, par exemple sur une surface, ou encore sur trois dimensions, par exemple sur un volume.

La mesure de l'augmentation d'au moins une dimension de l'élément gonflant 2 au cours du temps permet de déterminer, en fonction du temps, la quantité de sueur émise au niveau de la zone localisée de la peau 6 de l'individu ou de l'animal.

Les résultats obtenus sont, avantageusement, extrapolés pour être ramenés à une perte hydrique globale. L'extrapolation peut tenir compte, notamment, du rapport surfacique entre la zone localisée de la peau 6 recouverte par le dispositif et la surface totale de la peau 6 du corps. Elle peut également tenir compte de la position du capteur sur le corps humain, certaines zones du corps, notamment la cuisse, étant préférées pour le positionnement du capteur. L'extrapolation peut également prendre en compte les résultats d'un étalonnage, l'étalonnage pouvant être réalisé à partir d'une méthode de référence, telle la pesée. Le suivi de l'augmentation d'au moins une dimension permet d'évaluer, en temps réel et en continu, la perte hydrique de l'individu ou l'animal.

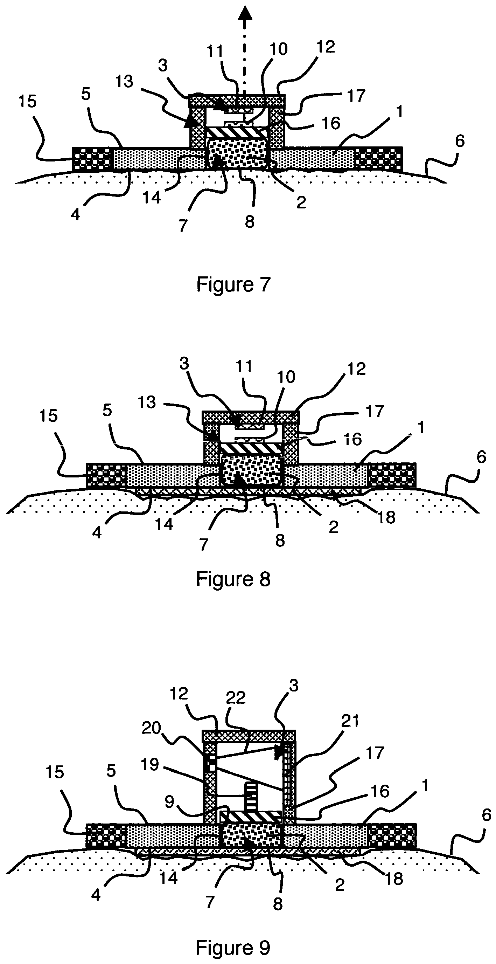

Selon un second mode de réalisation représenté aux figures 6 et 7, le dispositif est identique au premier mode de réalisation particulier décrit ci-dessus, à l'exception des moyens de confinement 13. Selon ce mode de réalisation particulier, les moyens de confinement 13 sont formés par une couche rigide ou semi-rigide 16 qui coopère avec des éléments de guidage 17. La couche rigide ou semi-rigide 16 est disposée au-dessus de l'élément gonflant 2 et entre les éléments de guidage 17. Les éléments de guidage 17 font saillie à la surface de la face de mesure 5. La couche rigide ou semi-rigide 16 est mobile le long desdits éléments 17.

Les éléments de guidage 17 peuvent être constitués par des rebords situés du côté de la face de mesure 5, autour de la seconde ouverture 9 et qui délimitent la couche rigide ou semi-rigide 16.

L'armature 12 peut comprendre les éléments de guidage 17 formant ainsi un seul ensemble. Alternativement, l'armature 12 peut être constituée par un couvercle posé sur les éléments de guidage 17 (en haut à la figure 6).

Comme représenté à la figure 7, sous l'effet de l'expansion de l'élément gonflant 2, la couche rigide ou semi-rigide 16 se déplace en translation selon une trajectoire d'expansion de l'élément gonflant 2 représentée par la flèche en pointillé.

Selon une variante non représentée, les éléments de guidage 17 peuvent comporter des rainures de type glissière dans lesquelles coulisse la couche rigide ou semi-rigide 16.

Selon une variante représentée à la figure 8, la face de contact 4 comporte une couche de drainage 18 de la sueur vers l'élément gonflant 2. Une fois le dispositif appliqué sur la zone localisée de la peau 6, la couche de drainage 18 est en contact avec la peau 6 et en communication fluidique avec l'élément gonflant 2. La sueur transpirée au niveau de cette zone localisée est drainée vers l'élément gonflant 2, par exemple, par capillarité ou grâce à un réseau fluidique formé par tout procédé connu. L'élément gonflant 2 n'est pas en contact direct avec la peau 6.

La couche de drainage 18 peut être composée d'une partie hydrophile qui va drainer la sueur jusqu'à l'élément gonflant 2. La couche de drainage 18 peut également comporter une partie hydrophobe, par exemple en cellulose ou en polyester, pour éviter l'absorption de la sueur au niveau de certaines zones de la face de contact 4.

Selon une variante non représentée, la couche de drainage 18 peut être constituée par une couche ayant une superficie plus grande que celle de la face de contact 4 de sorte que la couche de drainage 18 s'étend sur la peau 6 de l'individu ou de l'animal au-delà du support 1.

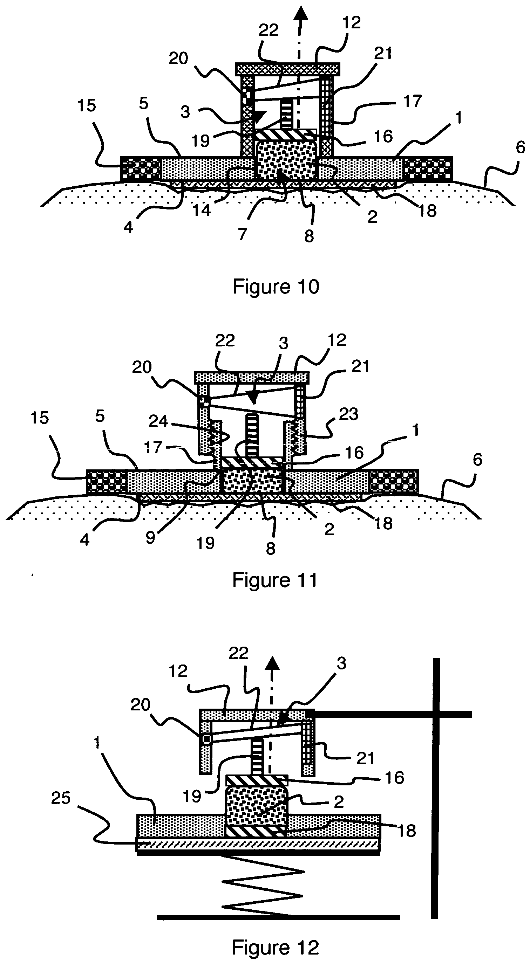

Selon un troisième mode de réalisation représenté aux figures 9 et 10, les moyens de mesure 3 comportent un cache optique 19 et un photodétecteur formé par une photodiode 20 et une fenêtre de détection 21. Selon ce mode particulier de réalisation, l'armature 12 comporte les éléments de guidage 17 et est destinée à la fois à supporter les moyens de mesure 3 et à guider la couche rigide ou semi-rigide 16. La photodiode 20 et la fenêtre de détection 21 sont situées sur la paroi latérale interne de l'armature 12. La fenêtre de détection 21 est en regard de la photodiode 20, sur le chemin optique du faisceau 22 émis par la photodiode 20.

Comme représenté à la figure 9, le cache optique 19 est solidaire de l'élément gonflant 2. Le cache optique 19 peut être fixé à la surface supérieure libre de la couche rigide ou semi-rigide 16 (en haut à la figure 9). Le cache optique 19 a une partie proéminente dépassant de la face de mesure 5. La partie proéminente est destinée à traverser le chemin optique du faisceau 22 émis par la photodiode 20.

Le signal électrique du photodétecteur varie proportionnellement à la quantité de lumière reçue du faisceau optique 22. L'augmentation de la dimension de l'élément gonflant 2 induit le déplacement du cache optique 19 en translation verticale (de bas en haut, à la figure 10). Ce déplacement amène le cache optique 19 entre la photodiode 20 et la fenêtre de détection 21, pour occulter progressivement le faisceau optique 22.

Comme représenté à la figure 10, lors de l'expansion de l'élément gonflant 2, la partie proéminente du cache optique 19 est positionnée entre la photodiode 20 et la fenêtre de détection 21, pour occulter au moins une partie du faisceau optique 22. L'intensité du signal électrique du photodétecteur est inversement proportionnelle à l'augmentation de la dimension de l'élément gonflant 2, dans le sens de la hauteur (de bas en haut aux figures 9 et 10) et, par conséquent, proportionnelle à la quantité de sueur absorbée par l'élément gonflant 2.

Selon une variante représentée à la figure 11, l'armature 12 comporte une partie ouverte inférieure 23 (en bas à la figure 11) qui s'ajuste à la partie supérieure 24 des éléments de guidage 17 (en haut à la figure 11). L'ajustement permet de solidariser l'armature 12 au support 1 et de positionner le cache optique 19 par rapport au photodétecteur. L'ajustement permet, notamment, de réaliser la mise à zéro du photodétecteur, avant l'application du dispositif d'évaluation sur la peau 6. La mise à zéro correspond au réglage à une valeur connue et reproductible de capacité ou de tension délivrée par le photodétecteur, avant utilisation des moyens de mesure 3. L'ajustement est réalisé par tout procédé connu.

À titre d'illustration représentée à la figure 11, la paroi interne de la partie ouverte inférieure 23 de l'armature 12 et la paroi externe de la partie supérieure 24 des éléments de guidage 17 sont filetées pour former un pas de vis et permettre de visser la partie ouverte inférieure 23 de l'armature 12 à la partie supérieure 24 des éléments de guidage 17. Le vissage ou dévissage de la partie ouverte inférieure 23 positionne le cache optique 19 verticalement. La valeur de la tension électrique recueillie à la sortie des moyens de mesure 3 est alors ajustée en fonction du chemin optique émis par la photodiode 20, par vissage ou dévissage de la partie ouverte inférieure 23 de l'armature 12, pour atteindre une valeur initiale de référence.

Cette variante peut être appliquée à des moyens de mesure 3 comportant un condensateur tel que décrit dans le second mode de réalisation. L'ajustement permet alors le positionnement de l'électrode fixe 11 par rapport à l'électrode mobile 10 pour obtenir une valeur initiale de référence.

Selon un quatrième mode de réalisation particulier non représenté, on mesure la déformation de l'élément gonflant 2 à l'aide d'un capteur de type jauge de contrainte. Par capteur de type jauge de contrainte, on entend un capteur mesurant une déformation mécanique à l'aide de moyens électriques. Dans ce cas, la jauge de contrainte peut être appliquée directement sur au moins une face libre de l'élément gonflant 2. La jauge de contrainte se présente, par exemple, sous la forme d'un serpentin en matériau conducteur, par exemple un matériau métallique ou un matériau polymère conducteur, dont on mesure la résistance électrique qui évolue au fur et à mesure que la jauge se déforme. La jauge de contrainte peut être formée directement sur l'élément gonflant 2 ou être rapportée sur l'élément gonflant 2, par exemple par collage, selon tout procédé connu. Le mode de réalisation est particulièrement avantageux car il permet de reporter l'ensemble du capteur directement sur l'élément gonflant 2 et de se dispenser d'un ajustement précis entre une partie fixe et une partie mobile. Par ailleurs, un tel mode de réalisation présente une conception plus simple que les modes de réalisation décrits précédemment et peut être réalisé à un coût moins élevé.

Le procédé d'évaluation peut également mettre en oeuvre des pratiques usuelles et courantes d'étalonnage. On entend par étalonnage, la réalisation au préalable d'une ou plusieurs courbes d'étalonnage dans des conditions similaires, pour une ou plusieurs températures comprises entre 35 °C et 40°C et notamment autour de 37 °C et pour des quantités connues de sueur ou d'un liquide comme l'eau ayant des propriétés physico-chimiques proches ou similaires à celles de la sueur. La ou les valeurs obtenues à partir du dispositif embarqué sur l'individu ou l'animal sont comparées aux courbes d'étalonnage.

### Exemple d'étalonnage

### Réalisation d'un dispositif d'étalonnage

Un dispositif, représenté à la figure 12, comporte un empilement de deux films de cellulose de 1 cm² de surface formant, respectivement, la couche de drainage 18 et la couche semi-rigide 16. Un mélange de 20 mg de polyacrylate et de coton forme l'élément gonflant 2 et est disposé entre les deux films de cellulose, 16 et 18. L'empilement formé est fixé sur une lame de verre 25 par un adhésif constituant le support 1. Un cache optique 19 constitué par un scotch en aluminium est collé sur la face libre du film de cellulose formant la couche semi-rigide 16.

Comme représenté à la figure 12, un photodétecteur commercialisé par la société OMROM, sous la référence EE-SG3, est placé au-dessus du cache optique 19 de sorte que ce dernier est disposé sur le chemin optique du faisceau 22 émis par le photodétecteur.

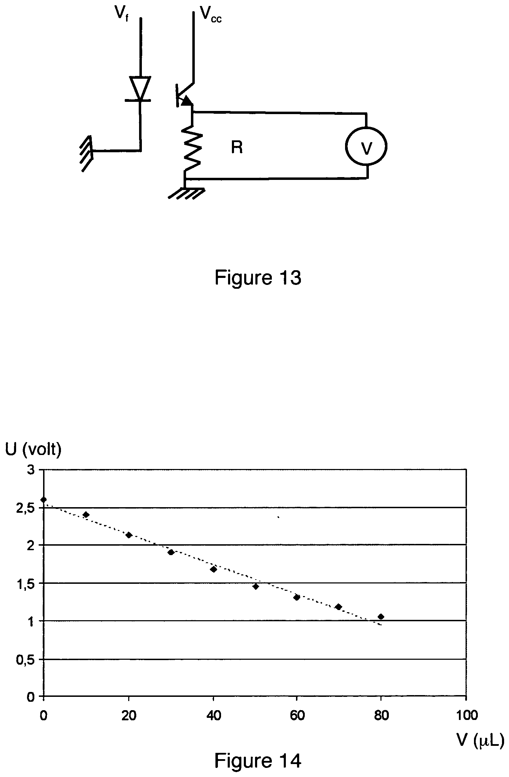

Un montage électronique, représenté à la figure 13, est réalisé afin de suivre la variation de la tension aux bornes d'une résistance, notée R, de 1 KΩ, avec les paramètres imposés suivants :
- V_{f} = 1.1 V et
- V_{cc} = 3V.

Une quantité d'eau prédéterminée, notée V, est déposée sur l'élément gonflant 2. On mesure ensuite la variation de la tension U aux bornes de la résistance R. Les résultats sont répertoriés dans le tableau suivant :

| **Volume d'eau absorbée en µL** | **Tension mesurée en Volt** |
|---|---|
| 0 | 2,6 |
| 10 | 2,4 |
| 20 | 2,13 |
| 30 | 1,9 |
| 40 | 1,67 |
| 50 | 1,45 |
| 60 | 1,3 |
| 70 | 1,18 |
| 80 | 1,05 |

La courbe d'étalonnage U=f(V) est représentée à la figure 14. On constate que la variation de la tension U mesurée aux bornes de la résistance R est proportionnelle à la quantité d'eau V absorbée par l'élément gonflant 2. La courbe d'étalonnage U=f(V) permet alors de déterminer la quantité de sueur absorbée sur un temps donné à partir d'une mesure de tension réalisée à partir d'un dispositif d'évaluation embarqué sur un individu ou un animal et similaire au dispositif d'étalonnage (même élément gonflant).

Le dispositif et le procédé d'évaluation selon l'invention permettent une évaluation en continu et en temps réel de la perte hydrique d'un individu ou d'un animal. L'utilisation du dispositif selon l'invention n'est pas limitée à un usage en laboratoire Le dispositif peut être utilisé par une personne étrangère au milieu hospitalier. En outre, le procédé d'évaluation est facile à mettre en oeuvre et donne des résultats fiables et sensibles permettant d'intervenir rapidement sur les causes de la déshydratation de l'individu ou de l'animal.

## Revendications

1. Dispositif d'évaluation de la perte hydrique d'un individu ou d'un animal par sudation, comportant :
- un support (1) souple, apte à pouvoir s'adapter à la forme du corps sur lequel il est appliqué, comportant une face de mesure (5) et une face de contact (4) destinée à être appliquée sur la peau (6) de l'individu ou de l'animal, **caractérisé en ce que** le dispositif est muni de:
- un élément gonflant (2) par absorption de sueur et solidaire du support (1), ledit élément gonflant (2) étant agencé pour être en contact direct ou en communication fluidique avec la peau (6) et,
- des moyens de mesure (3) de l'augmentation d'au moins une dimension de l'élément gonflant (2), ladite augmentation étant provoquée par absorption de ladite sueur.

2. Dispositif selon la revendication 1, **caractérisé en ce que** l'élément gonflant (2) comporte un polymère hydrophile.

3. Dispositif selon la revendication 2, **caractérisé en ce que** le polymère hydrophile est choisi parmi les polymères hydrophiles synthétiques, les polysaccharides naturels, les polysaccharides hémi-synthétiques et les dérivés de la cellulose et leurs sels.

4. Dispositif selon l'une des revendications 2 et 3, **caractérisé en ce que** le polymère hydrophile est choisi parmi les polyvinylpyrrolidones, les polymères dérivés des acides acrylique et méthylacrylique et leurs sels, et les polymères d'acides aminés.

5. Dispositif selon l'une quelconque des revendications 1 à 4, **caractérisé en ce que** l'élément gonflant (2) comporte des agents de stimulation du gonflement dudit élément (2).

6. Dispositif selon l'une quelconque des revendications 1 à 5, **caractérisé en ce que** les moyens de mesure (3) sont choisis parmi les photodétecteurs, les capteurs capacitifs, les capteurs inductifs, les capteurs mécaniques de position et les capteurs de type jauge de contrainte.

7. Dispositif selon l'une quelconque des revendications 1 à 6, **caractérisé en ce qu'**il comporte une armature (12) située sur la face de mesure (5) et **en ce qu'**au moins une partie des moyens de mesure (3) est supportée par ladite armature (12).

8. Dispositif selon l'une quelconque des revendications 1 à 7, **caractérisé en ce que** la face de contact (4) comporte une couche de drainage (18) de la sueur vers l'élément gonflant (2).

9. Dispositif selon l'une quelconque des revendications 1 à 8, **caractérisé en ce que** le support (1) comporte un trou traversant (7) débouchant sur la face de contact (4) et sur la face de mesure (5) dans lequel est logé l'élément gonflant (2) et **en ce que** les moyens de mesure (3) sont disposés au niveau de la face de mesure (5).

10. Dispositif selon la revendication 9, caractérisé en ce **caractérisé en ce que** l'élément gonflant (2) est logé et contraint dans le trou traversant (7) de sorte que l'augmentation d'une dimension de l'élément gonflant (2) provoque la saillie dudit élément (2) hors du trou traversant (7) et **en ce que** les moyens de mesure (3) sont disposés au-dessus du trou traversant (7).

11. Dispositif selon la revendication 10, **caractérisé en ce que** la face de mesure (5) comporte des moyens de confinement (13) de l'élément gonflant (2) faisant saillie hors du trou traversant (7).

12. Dispositif selon la revendication 11, **caractérisé en ce que** les moyens de confinement (13) sont formés par une membrane déformable fixée à la face de mesure (5) et recouvrant au moins une partie de l'élément gonflant (2).

13. Dispositif selon la revendication 11, **caractérisé en ce que** les moyens de confinement (13) sont formés par une couche rigide ou semi-rigide (16) disposée au-dessus de l'élément gonflant (2) qui coopère avec des éléments de guidage (17) faisant saillie à la surface de la face de mesure (5) et **en ce que** ladite couche (16) est mobile le long des éléments de guidage (17).

14. Procédé d'évaluation de la perte hydrique d'un individu ou d'un animal par sudation, **caractérisé en ce qu'**on applique un dispositif d'évaluation selon l'une quelconque des revendications 1 à 13, sur une zone localisée de la peau (6) de l'individu ou l'animal, la face de contact (4) étant positionnée en regard de la peau (6) et l'élément gonflant (2) étant en contact ou en communication fluidique avec la peau (6) et **en ce qu'**on mesure ensuite l'augmentation d'au moins une dimension de l'élément gonflant (2) au cours du temps grâce aux moyens de mesure (3), tout en maintenant ledit dispositif en place.

15. Procédé selon la revendication 14, **caractérisé en ce qu'**on mesure l'augmentation d'une seule dimension de l'élément gonflant (2) au cours du temps.

16. Procédé selon l'une des revendications 14 et 15, **caractérisé en ce que** la face de contact (4) comporte une couche de drainage (18) de la sueur et **en ce qu'**une fois le dispositif appliqué sur la zone localisée de la peau (6), la couche de drainage (18) est en contact avec la peau (6) et en communication fluidique avec l'élément gonflant (2).

## Patentansprüche

1. Vorrichtung zur Abschätzung des Wasserverlustes einer Person oder eines Tieres durch Schwitzen, mit:
- einer weichen Auflage (1), die dazu geeignet ist, sich an die Form des Körpers anzupassen, auf den sie aufgebracht wird, mit einer Messfläche (5) und einer Anlagefläche (4), die zum Aufbringen auf die Haut (6) der Person oder des Tieres vorgesehen ist,
**dadurch gekennzeichnet, dass** die Vorrichtung Folgendes umfasst:
- ein Element (2), welches durch die Aufnahme von Schweiß quillt und mit der Auflage (1) fest verbunden ist, wobei das Quellelement (2) so eingerichtet ist, dass es in unmittelbarem Kontakt oder in Fließverbindung mit der Haut (6) steht, und
- Einrichtungen zum Messen (3) der Vergrößerung von mindestens einer Abmessung des Quellelementes (2), wobei die Vergrößerung durch Aufnahme des Schweißes bewirkt wird.

2. Vorrichtung nach Anspruch 1, **dadurch gekennzeichnet, dass** das Quellelement (2) ein hydrophiles Polymer aufweist.

3. Vorrichtung nach Anspruch 2, **dadurch gekennzeichnet, dass** das hydrophile Polymer aus den synthetischen hydrophilen Polymeren, den natürlichen Polysacchariden, den halbsynthetischen Polysacchariden und den Derivaten der Zellulose und ihren Salzen ausgewählt ist.

4. Vorrichtung nach einem der Ansprüche 2 und 3, **dadurch gekennzeichnet, dass** das hydrophile Polymer aus den Polyvinylpyrrolidonen, den aus Acryl- und Methacrylsäure und ihren Salzen abgeleiteten Polymeren sowie den Polymeren von Aminosäuren ausgewählt ist.

5. Vorrichtung nach einem der Ansprüche 1 bis 4, **dadurch gekennzeichnet, dass** das Quellelement (2) Wirkstoffe zur Stimulierung des Quellens des Elementes (2) aufweist.

6. Vorrichtung nach einem der Ansprüche 1 bis 5, **dadurch gekennzeichnet, dass** die Messeinrichtungen (3) aus den Fotodetektoren, den kapazitiven Sensoren, den induktiven Sensoren, den mechanischen Positionssensoren und den Sensoren vom Typ eines Dehnungsmessstreifens ausgewählt sind.

7. Vorrichtung nach einem der Ansprüche 1 bis 6, **dadurch gekennzeichnet, dass** sie einen auf der Messfläche (5) angeordneten Anker (12) aufweist und dass zumindest ein Teil der Messeinrichtungen (3) von dem Anker (12) abgestützt wird.

8. Vorrichtung nach einem der Ansprüche 1 bis 7, **dadurch gekennzeichnet, dass** die Anlagefläche (4) eine Ablaufschicht (18) des Schweißes in Richtung des Quellelementes (2) aufweist.

9. Vorrichtung nach einem der Ansprüche 1 bis 8, **dadurch gekennzeichnet, dass** die Auflage (1) eine Durchgangsöffnung (7) aufweist, die in die Anlagefläche (4) und in die Messfläche (5) mündet, in der sich das Quellelement (2) befindet, und dass die Messeinrichtungen (3) auf der Höhe der Messfläche (5) angeordnet sind.

10. Vorrichtung nach Anspruch 9, **dadurch gekennzeichnet, dass** das Quellelement (2) in der Durchgangsöffnung (7) derart untergebracht und eingeklemmt ist, dass die Vergrößerung einer Abmessung des Quellelementes (2) den Überstand des Elementes (2) über die Durchgangsöffnung (7) hinaus bewirkt, und dass die Messeinrichtungen (3) oberhalb der Durchgangsöffnung (7) angeordnet sind.

11. Vorrichtung nach Anspruch 10, **dadurch gekennzeichnet, dass** die Messfläche (5) Begrenzungseinrichtungen (13) des Quellelementes (2) aufweist, die über die Durchgangsöffnung (7) hervorstehen.

12. Vorrichtung nach Anspruch 11, **dadurch gekennzeichnet, dass** die Begrenzungseinrichtungen (13) aus einer verformbaren Membran bestehen, die an der Messfläche (5) befestigt ist und zumindest einen Teil des Quellelementes (2) abdeckt.

13. Vorrichtung nach Anspruch 11, **dadurch gekennzeichnet, dass** die Begrenzungseinrichtungen (13) aus einer über dem Quellelement (2) angeordneten, starren oder halbstarren Schicht (16) bestehen, welche mit Leitelementen (17) zusammenwirkt, die aus der Oberfläche der Messfläche (5) hervorstehen, und dass die Schicht (16) entlang der Leitelemente (17) beweglich ist.

14. Verfahren zur Abschätzung des Wasserverlustes einer Person oder eines Tieres durch Schwitzen, **dadurch gekennzeichnet, dass** eine Abschätzvorrichtung gemäß einem der Ansprüche 1 bis 13 auf einen lokal begrenzten Bereich der Haut (6) der Person oder des Tieres aufgebracht wird, wobei die Anlagefläche (4) gegenüber der Haut (6) angebracht wird und das Quellelement (2) in Kontakt oder in Fließverbindung mit der Haut (6) steht, und dass sodann die Vergrößerung von mindestens einer Abmessung des Quellelementes (2) über die Zeit mit Hilfe der Messeinrichtungen (3) gemessen wird, während die Vorrichtung gleichzeitig an ihrem Platz gehalten wird.

15. Verfahren nach Anspruch 14, **dadurch gekennzeichnet, dass** die Vergrößerung einer einzigen Abmessung des Quellelementes (2) über die Zeit gemessen wird.

16. Verfahren nach einem der Ansprüche 14 und 15, **dadurch gekennzeichnet, dass** die Anlagefläche (4) eine Ablaufschicht (18) des Schweißes aufweist und dass, sobald die Vorrichtung auf den lokal begrenzten Bereich der Haut (6) aufgebracht worden ist, die Ablaufschicht (18) in Kontakt mit der Haut (6) und in Fließverbindung mit dem Quellelement (2) steht.

## Claims

1. A device for evaluating the hydric loss of a person or an animal by sweating, comprising :
- a flexible support (1) able to adapt to the shape of the body on which the support is applied, comprising a measuring face (5) and a contact face (4) designed to be applied to the skin (6) of the person or animal, **characterized in that** the device is provided with:
- a swelling element (2), securedly affixed to the support (1), that swells by absorption of sweat, said swelling element (2) being arranged to be in direct contact or in fluidic communication with the skin (6) and,
- measuring means (3) for measuring the increase of at least one dimension of the swelling element (2), said increase being caused by absorption of said sweat.

2. The device according to claim 1, **characterized in that** the swelling element (2) comprises a hydrophilic polymer.

3. The device according to claim 2, **characterized in that** the hydrophilic polymer is chosen from synthetic hydrophilic polymers, natural polysaccharides, semi-synthetic polysaccharides and derivatives of cellulose and their salts.

4. The device according to one of claims 2 and 3, **characterized in that** the hydrophilic polymer is chosen from polyvinylpyrrolidones, polymers derived from acrylic and methylacrylic acids and their salts, and amino acid polymers.

5. The device according to any one of claims 1 to 4, **characterized in that** the swelling element (2) comprises swelling stimulation agents of said element (2).

6. The device according to any one of claims 1 to 5, **characterized in that** the measuring means (3) are chosen from photodetectors, capacitive sensors, inductive sensors, mechanical position sensors and sensors of strain gage type.

7. The device according to any one of claims 1 to 6, **characterized in that** it comprises an armature (12) situated on the measuring face (5) and **in that** at least a part of the measuring means (3) are supported by said armature (12).

8. The device according to any one of claims 1 to 7, **characterized in that** the contact face (4) comprises a drainage layer (18) of the sweat to the swelling element (2).

9. The device according to any one of claims 1 to 8, **characterized in that** the support (1) comprises a through hole (7) opening out onto the contact face (4) and onto the measuring face (5) in which the swelling element (2) is housed and **in that** the measuring instrument (3) is arranged at the level of the measuring face (5).

10. The device according to claim 9, **characterized in that** the swelling element (2) is housed and stressed in the through hole (7) so that an increase of a dimension of the swelling element (2) makes said element (2) protrude out from the through hole (7) and **in that** the measuring means (3) are arranged above the through hole (7).

11. The device according to claim 10, **characterized in that** the measuring face (5) comprises confinement means (13) of the swelling element (2) salient from the through hole (7).

12. The device according to claim 11, **characterized in that** the confinement means (13) are formed by a deformable membrane fixed to the measuring face (5) and covering at least a part of the swelling element (2).

13. The device according to claim 11, **characterized in that** the confinement means (13) are formed by a rigid or semi-rigid layer (16) arranged above the swelling element (2) which operates in conjunction with guiding elements (17) salient from the surface of the measuring face (5) and **in that** said layer (16) is mobile along the guiding elements (17).

14. A method for evaluating the hydric loss of a person or an animal by sweating, **characterized in that** an evaluation device according to any one of claims 1 to 13 is applied on a localized area of the skin (6) of the person or animal, the contact face (4) being positioned facing the skin (6) and the swelling element (2) being in contact or in fluidic communication with the skin (6) and **in that** the increase of at least one dimension of the swelling element (2) is then measured over time by means of the measuring means (3), while at the same time keeping said device in place.

15. The method according to claim 14, **characterized in that** the increase of a single dimension of the swelling element (2) is measured over time.

16. The method according to one of claims 14 and 15, **characterized in that** the contact face (4) comprises a drainage layer (18) of the sweat and **in that**, once the device has been applied on the localized area of the skin (6), the drainage layer (18) is in contact with the skin (6) and in fluidic communication with the swelling element (2).
